# EUROPÄISCHE PATENTSCHRIFT

(11) **EP 0 781 770 B1**
(45) Veröffentlichungstag und Bekanntmachung des Hinweises auf die Patenterteilung: **05.03.2003**
(21) Anmeldenummer: 96119495.8
(22) Anmeldetag: 05.12.1996
(51) Int. Cl.: C07D 319/06, C09K 19/34

(54) **1,3-Dioxane und flüssigkristallines Medium**
1,3-Dioxanes and liquid crystalline medium
1,3-Dioxanes et milieu de cristaux liquides

(30) Priorität: 29.12.1995 DE 19549123
(43) Veröffentlichungstag der Anmeldung: 02.07.1997
(73) Patentinhaber: MERCK PATENT GmbH, 64293 Darmstadt (DE)
(72) Erfinder: Poetsch, Eike, Dr., 64367 Mühltal (DE); Lannert, Harald, 64407 Fränkisch Crumbach (DE); Krause, Joachim, Dr., 64807 Dieburg (DE); Tarumi, Kazuaki, Dr., 64342 Seeheim (DE)

(56) Entgegenhaltungen:
- EP-A- 0 154 840
- EP-A- 0 464 648
- WO-A-86/03769
- WO-A-88/02018
- WO-A-94/06885

## Beschreibung

Die Erfindung betrifft 1,3-Dioxane der allgemeinen Formel l, 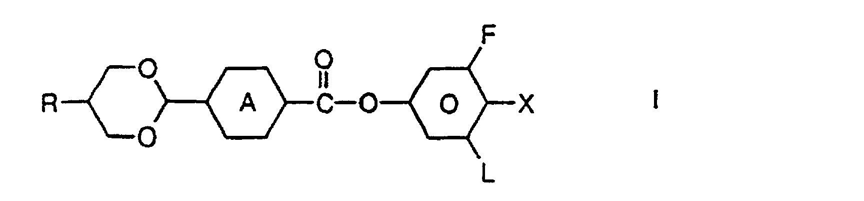 worin
- R: H, einen unsubstituierten, einen einfach durch CN oder CF₃ oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, 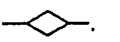 -CO-, -CO-O-, -O-CO- oder -O-CO-O- so ersetzt sein können, daß O-Atome nicht direkt miteinander verknüpft sind, 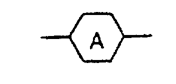
(a) trans-1,4-Cyclohexylen.
(b) 1,4-Phenylen, worin auch ein oder zwei CH-Gruppen durch N ersetzt sein können,
(c) 1,4-Cyclohexenylen,
wobei die Reste (b) und (c) ein- oder mehrfach durch Fluor substituiert sein können.
- X: F, Cl, halogeniertes Alkyl, Alkoxy oder Alkenyl mit 1-5 C-Atomen, und
- L: H oder F
bedeuten,
mit der Maßgabe, daß
im Fall X = F oder X = Cl L Fluor bedeutet.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindung der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristaltine oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über vergleichsweise niedrige Viskositäten. Mit ihrer Hilfe lassen sich stabile flüssigkristalline Medien mit breitem Mesophasenbereich und vorteilhaften Werten für die optische und dielektrische Anisotropie erhalten. Diese Medien weisen ferner ein sehr gutes Tieftemperaturverhalten auf.

1,3-Dioxane der Formel I, worin X = L= F bedeuten, werden in der EP 0 387 032 von der allgemeinen Formel erfaßt, aber nicht genannt.

Aus der EP 0 447 565 sind 1,3-Dioxane der Formel 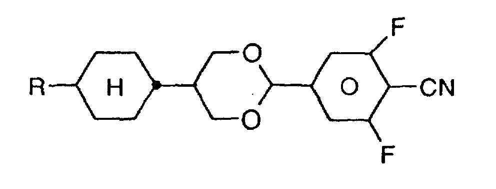 sowie deren Verwendung in STN-Mischungen bekannt.

Aus der WO 86/03769 und WO 88/02018 sind Dioxan-Derivate bekannt, die einen 3,4-Difluorbenzolring enthalten.

In der EP 0 464 648 A1 werden Dioxan-Derivate mit einem terminalen Nitrilrest beschrieben.

In der U.S. 5,322,638 werden 1,3-Dioxane der Formel 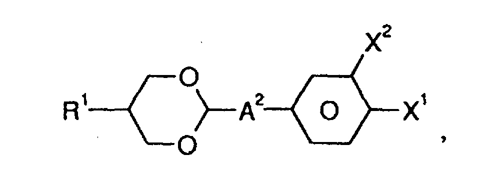 wobei R¹ ein Alkenylrest, A² ein 1,4-Phenylring oder trans-1,4-Cyclohexylenring, X¹ F oder Cl und X² Fluor bedeutet, beansprucht.

Aus der EP 0 400 861 sind Phenyldioxane der Formel 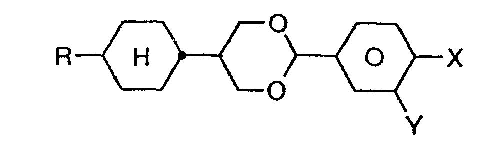 X = CN oder F
Y = H oder F
bekannt.

Im Hinblick auf die verschiedensten Einsatzbereiche der 1,3-Dioxane mit hohem Δε war es jedoch wünschenswert, weitere Verbindungen mit hoher Nematogenität zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren. Die erfindungsgemäßen Verbindungen zeichnen sich insbesondere durch ihren breiten Mesophasenbereich bei guten Viskositätseigenschaften und Δε-Werten aus.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektrooptische Anzeigeelemente, die derartige Medien enthalten.

Bevorzugte Verbindungen der Formel I sind diejenigen der Teilformeln I1 bis I5: 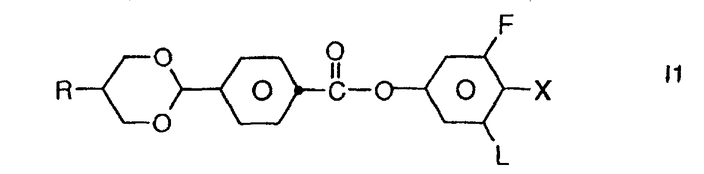 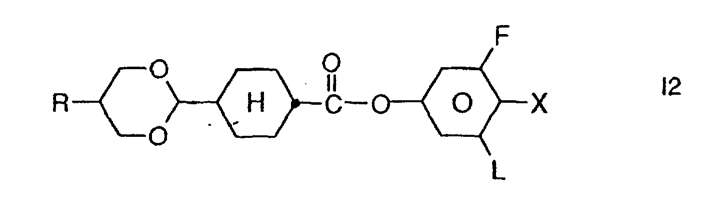 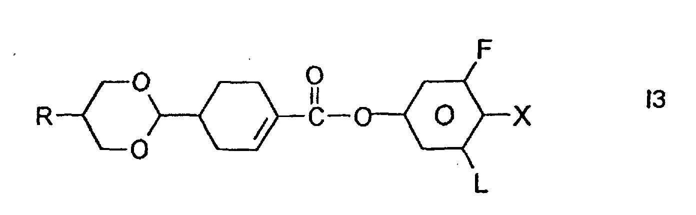 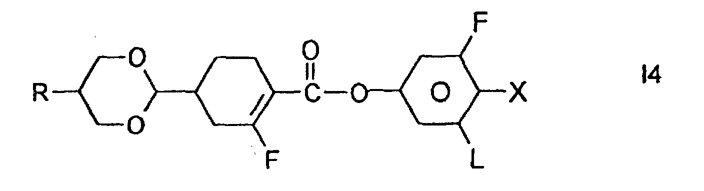 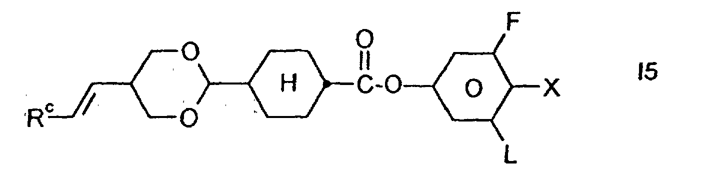 R^{c} = H, CH₃, C₂H₅ oder n-C₃H₇

In den Verbindungen der Formel I bedeutet R vorzugsweise geradkettiges Alkyl oder Alkenyl mit 1-5 C-Atomen. Der Alkenylrest ist vorzugsweise 1 E-Alkenyl, 3E-Alkenyl oder 4-Alkenyl.

L ist vorzugsweise F.

X bedeutet vorzugsweise F, Cl, OCF₃, OCHF₂, CF₃, CHF₂, OCHFCF₃, OCHFCHF₂, OCH₂CF₃, OC₂F₅, OC₃F₇, OCH=CHF, OCF=CHF, OCH=CF₂, OCF = CF₂, OCF₂CHFCF₃, insbesondere F, OCF₃, OCHF₂, OCHFCF₃, OCHFCHF₂ und OCH=CF₂.

Insbesondere bevorzugt sind Verbindungen der Formel I, worin X = L = F ist.

Falls R in den Verbindungen der Formel I einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3-oder Pent-4-enyl, Hex-1-, 2-, 3-, 4- oder Hex-5-enyl, Hept-1-, 2-, 3-, 4-, 5-oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonyl-gruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl; Pentoxycarbonyl, Methoxycarbonylmethyl, Ethoxy-carbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch unsubstituiertes oder substituiertes -CH=CH- und eine benachbarte CH₂-Gruppe durch CO oder CO-O oder O-CO- ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacyyloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I, die über für Polymerisationsreaktionen geeignete Flügelgruppen R verfügen, eignen sich zur Darstellung flüssigkristalliner Polymerer.

Verbindungen der Formel I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3.3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxy-carbonyl)-heptyl, 8,8-Bis(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optische Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

Die Verbindungen der Formel l werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Die Verbindungen sind einfach zugänglich, indem der 1,3-Dioxanring durch Wasserabspaltung in der Reaktion des entsprechenden Diols mit dem Aldehyd gebildet wird bzw. daß nach literaturbekannten Verfahren der Substituent X direkt über das entsprechende Wasserstoff-, OH-, Tosylat-, Triflat-, Brom-, Jod-, Metall- oder Aldehydderivat gebildet wird.

Die erfindungsgemäßen Verbindungen lassen sich z.B. wie folgt herstellen:

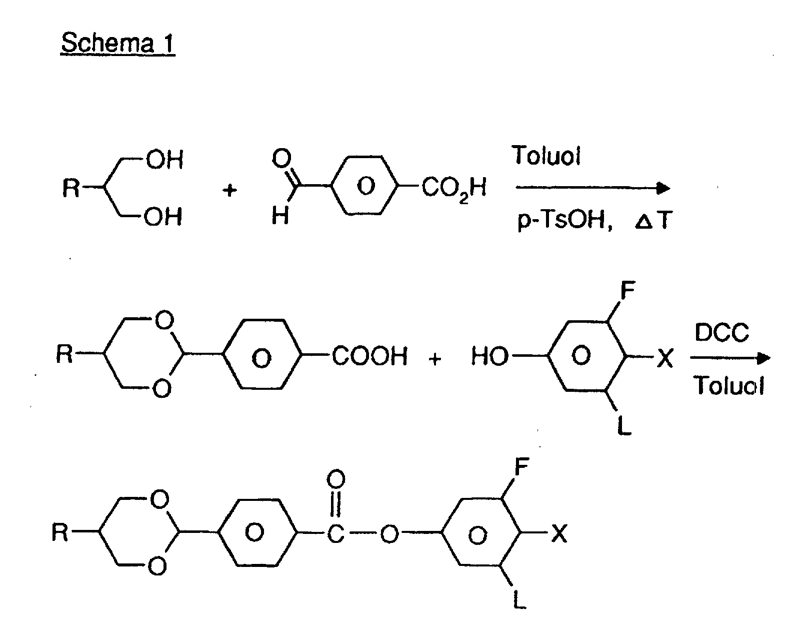

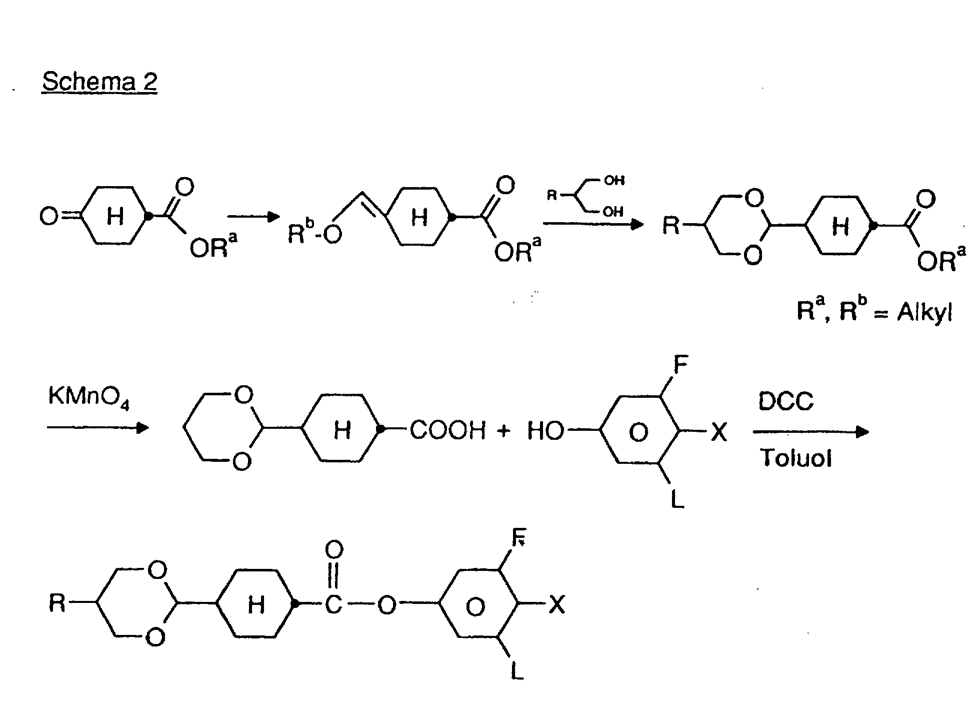

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexane, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2-5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢCH₃₋₍ₖ₊ₗ₎FₖClₗ, wobei i 0 oder 1 und k+l 1, 2 oder 3 sind; die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 10 bis 90 %, insbesondere 30 bis 90 %
- Gruppe B:: 0 bis 80 %, vorzugsweise 10 bis 80 %, insbesondere 10 bis 65%
- Gruppe C:: 0 bis 80%, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 bis 90 % und insbesondere 10 bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nC | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-C₂H₂ₛ- | CN | H | H |
| nAm | CₙH₂ₙ₊₁ | COOCₘH₂ₘ₊₁ | H | H |

Erfindungsgemäße Mischungen enthalten neben ein oder mehreren Verbindungen der Formel l insbesondere Verbindungen ausgewählt aus den Tabellen A und B.

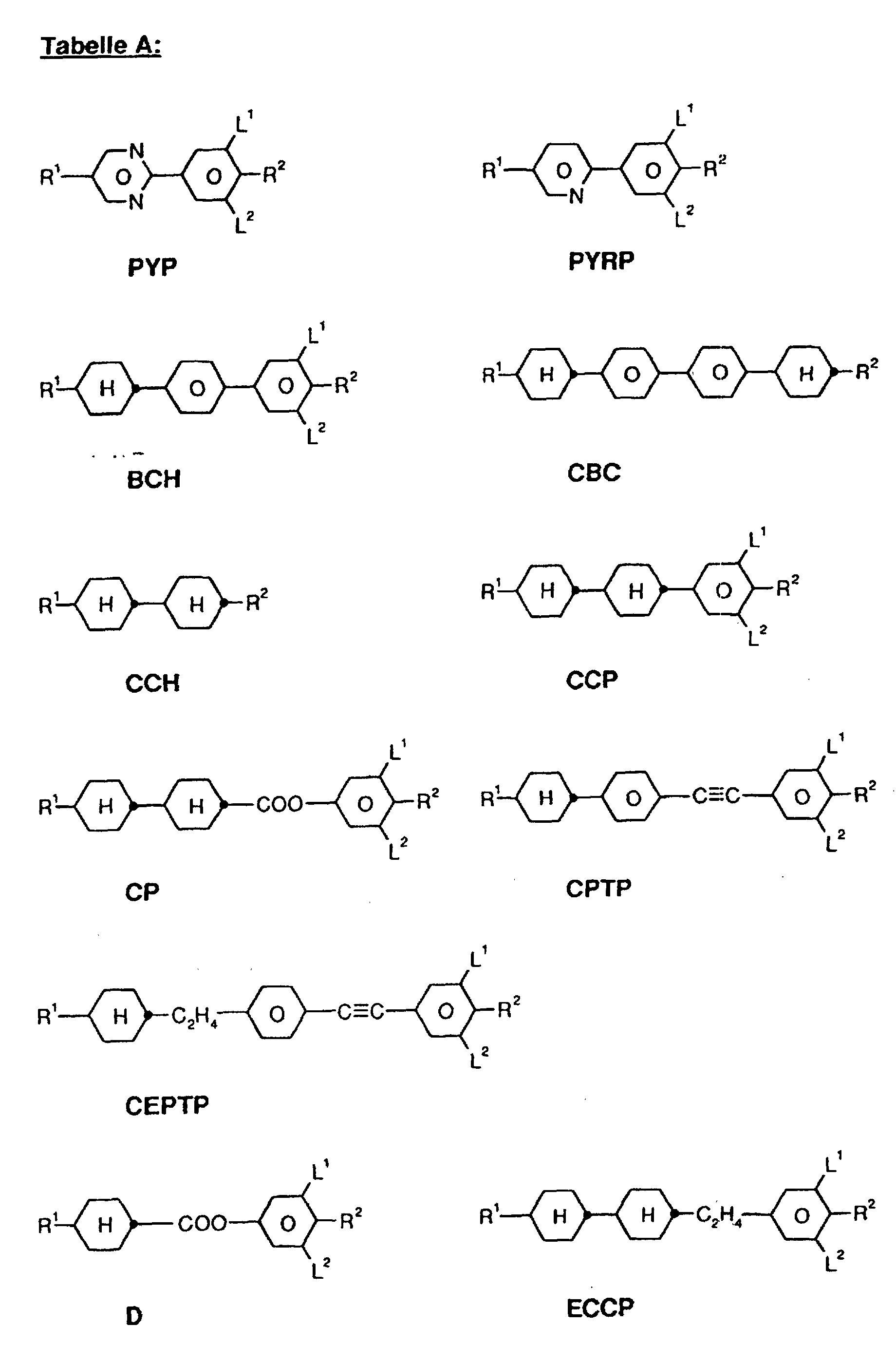

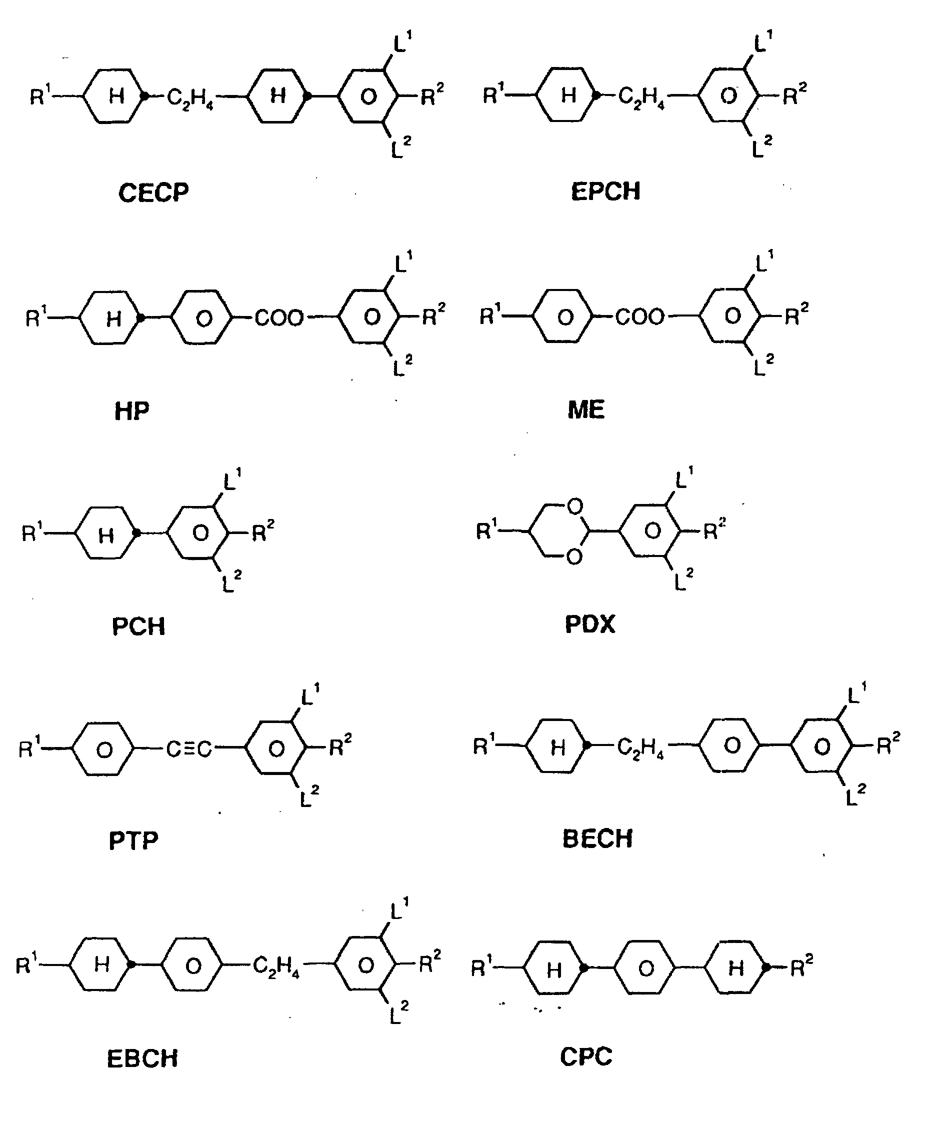

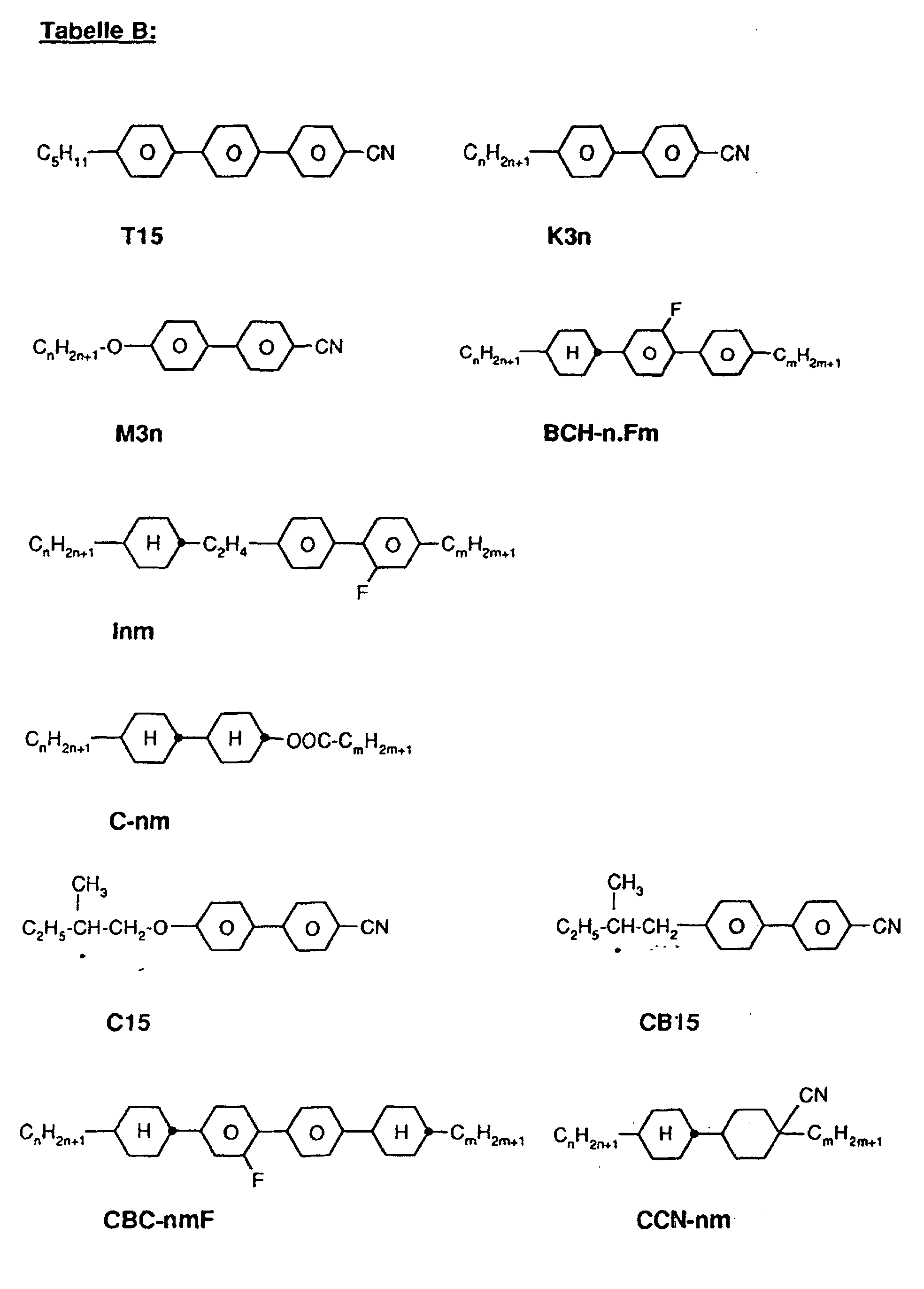

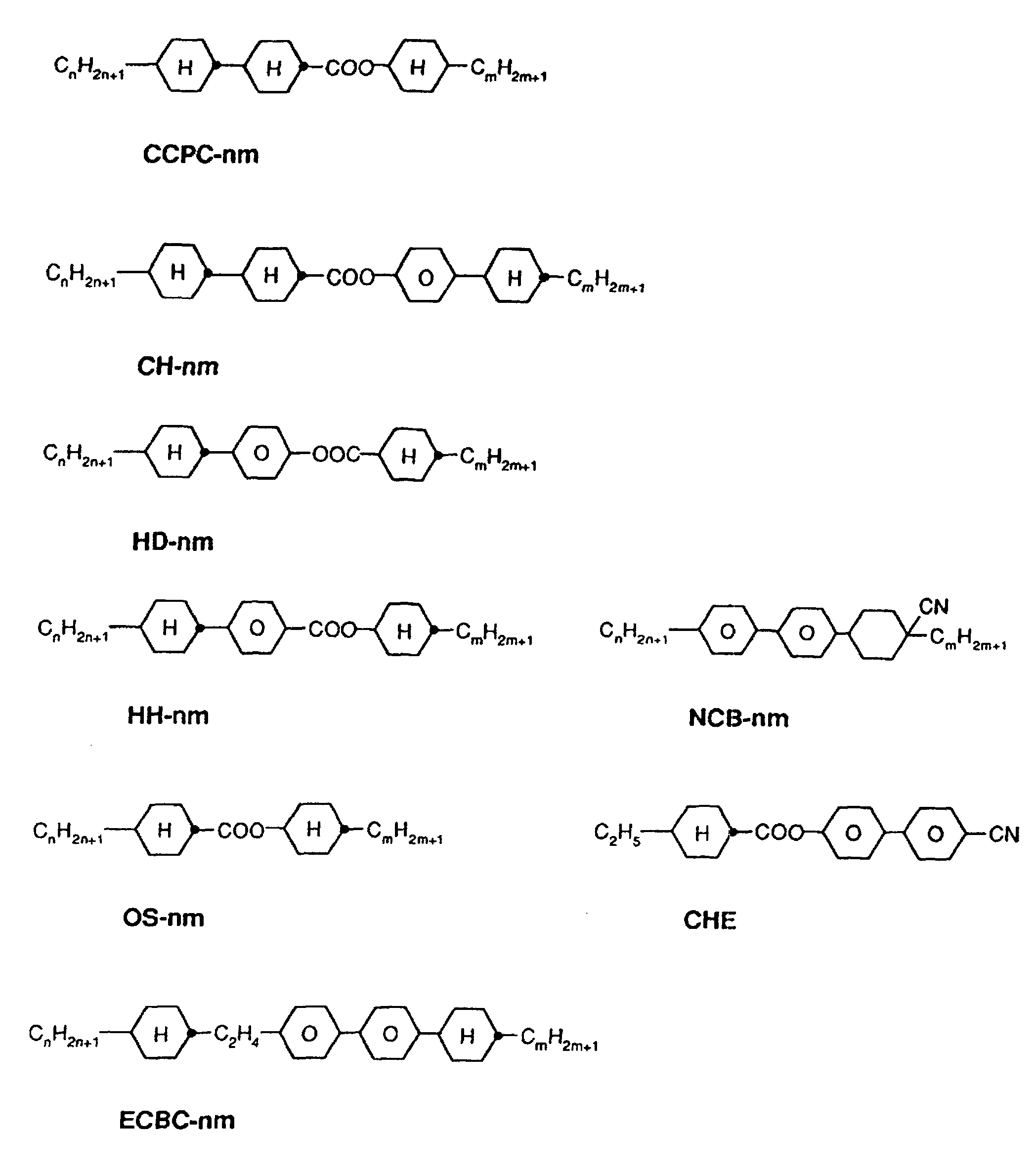

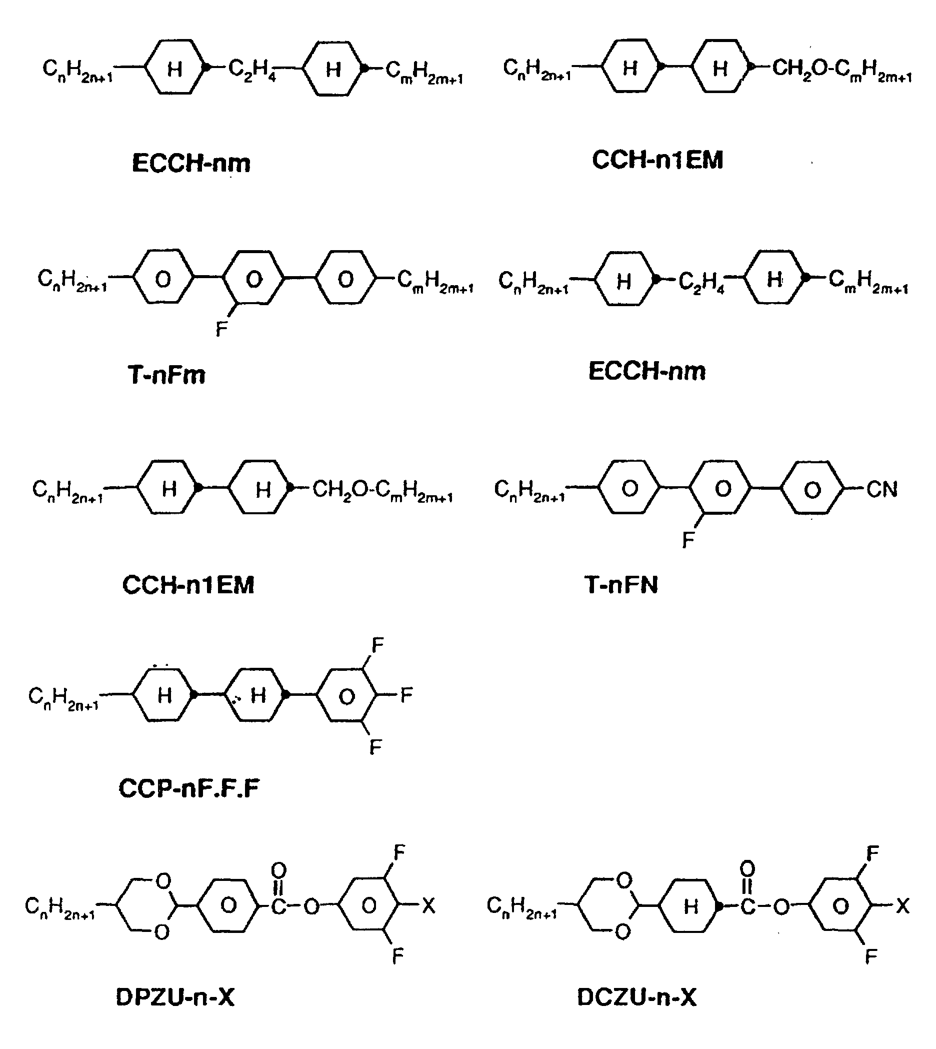

Die folgenden Beispiele sollen die Erfindung erläutern. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Dichlormethan, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DCC: N,N'-Dicyclohexylcarbodiimid
- DMAP: 4-(Dimethylamino)-pyridin
- KOT: Kalium-tertiär-butanolat
- THF: Tetrahydrofuran
- pTsOH: p-Toluolsulfonsäure

### Beispeil 1

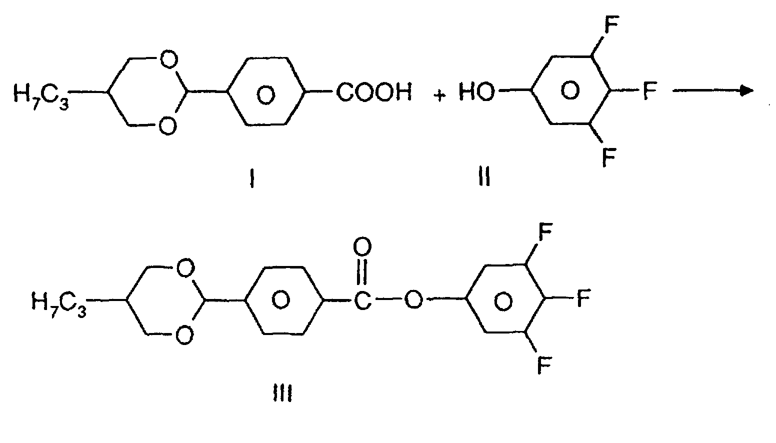

55 mmol 3,4,5-Trifluorphenol in 75 ml Toluol und 55 mmol DCC werden 1 h bei Raumtemperatur gerührt. Nach Zugabe von 50 mmol I wird über Nacht bei Raumtemperatur gerührt. Anschließend versetzt man das Gemisch mit 1 g Oxalsäure und rührt weitere zwei Stunden bei Raumtemperatur. Zuletzt wird wie üblich aufgearbeitet. Das Produkt wird in Toluol/Hexan (1:1) umkristallisiert. K 111 N (96,7) l.

Analog werden die folgenden Verbindungen der Formel 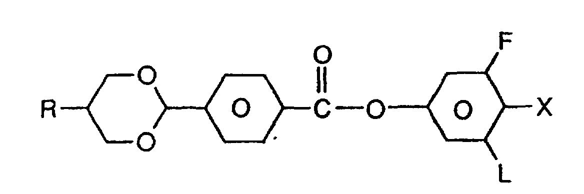 hergestellt:

| R | X | L | |
|---|---|---|---|
| CH₃ | F | H | |
| CH₃ | F | F | |
| C₂H₅ | F | H | |
| C₂H₅ | F | F | |
| n-C₃H₇ | F | H | |
| n-C₄H₉ | F | H | |
| n-C₄H₉ | F | F | |
| n-C₅H₁₁ | F | H | |
| n-C₅H₁₁ | F | F | K 94 S_{A} (92) N 100,2 l; Δn = +0,105; Δε = 30,65 |
| n-C₆H₁₃ | F | H | |
| n-C₆H₁₃ | F | F | |
| CH₃ | Cl | H | |
| CH₃ | Cl | F | |
| C₂H₅ | Cl | H | |
| C₂H₅ | Cl | F | |
| n-C₃H₇ | Cl | H | |
| n-C₃H₇ | Cl | F | |
| n-C₄H₉ | Cl | H | |
| n-C₄H₉ | Cl | F | |
| n-C₅H₁₁ | Cl | H | |
| n-C₅H₁₁ | Cl | F | |
| n-C₆H₁₃ | Cl | H | |
| n-C₆H₁₃ | Cl | F | |
| CH₃ | OCF₃ | H | |
| CH₃ | OCF₃ | F | |
| C₂H₅ | OCF₃ | H | |
| C₂H₅ | OCF₃ | F | |
| n-C₃H₇ | OCF₃ | H | |
| n-C₃H₇ | OCF₃ | F | |
| n-C₄H₉ | OCF₃ | H | |
| n-C₄H₉ | OCF₃ | F | |
| n-C₅H₁₁ | OCF₃ | H | |
| n-C₅H₁₁ | OCF₃ | F | |
| n-C₆H₁₃ | OCF₃ | H | |
| n-C₆H₁₃ | OCF₃ | F | |
| CH₃ | CF₃ | H | |
| CH₃ | CF₃ | F | |
| C₂H₅ | CF₃ | H | |
| C₂H₅ | CF₃ | F | |
| n-C₃H₇ | CF₃ | H | |
| n-C₃H₇ | CF₃ | F | |
| n-C₄H₉ | CF₃ | H | |
| n-C₄H₉ | CF₃ | F | |
| n-C₅H₁₁ | CF₃ | H | |
| n-C₅H₁₁ | CF₃ | F | |
| n-C₆H₁₃ | CF₃ | H | |
| n-C₆H₁₃ | CF₃ | F | |
| CH₃ | CF₂H | H | |
| CH₃ | CF₂H | F | |
| C₂H₅ | CF₂H | H | |
| C₂H₅ | CF₂H | F | |
| n-C₃H₇ | CF₂H | H | |
| n-C₃H₇ | CF₂H | F | |
| n-C₄H₉ | CF₂H | H | |
| n-C₄H₉ | CF₂H | F | |
| n-C₅H₁₁ | CF₂H | H | |
| n-C₅H₁₁ | CF₂H | F | |
| n-C₆H₁₃ | CF₂H | H | |
| n-C₆H₁₃ | CF₂H | F | |
| CH₃ | OCH₂CF₃ | H | |
| CH₃ | OCH₂CF₃ | F | |
| C₂H₅ | OCH₂CF₃ | H | |
| C₂H₅ | OCH₂CF₃ | F | |
| n-C₃H₇ | OCH₂CF₃ | H | |
| n-C₃H₇ | OCH₂CF₃ | F | |
| n-C₄H₉ | OCH₂CF₃ | H | |
| n-C₄H₉ | OCH₂CF₃ | F | |
| n-C₅H₁₁ | OCH₂CF₃ | H | |
| n-C₅H₁₁ | OCH₂CF₃ | F | |
| n-C₆H₁₃ | OCH₂CF₃ | H | |
| n-C₆H₁₃ | OCH₂CF₃ | F | |
| CH₃ | OCH=CF₂ | H | |
| CH₃ | OCH=CF₂ | F | |
| C₂H₅ | OCH=CF₂ | H | |
| C₂H₅ | OCH=CF₂ | F | |
| n-C₃H₇ | OCH=CF₂ | H | |
| n-C₃H₇ | OCH=CF₂ | F | |
| n-C₄H₉ | OCH=CF₂ | H | |
| n-C₄H₉ | OCH=CF₂ | F | |
| n-C₅H₁₁ | OCH=CF₂ | H | |
| n-C₅H₁₁ | OCH=CF₂ | F | |
| n-C₆H₁₃ | OCH=CF₂ | H | |
| n-C₆H₁₃ | OCH=CF₂ | F | |
| CH₃ | OCHFCF₃ | H | |
| CH₃ | OCHFCF₃ | F | |
| C₂H₅ | OCHFCF₃ | H | |
| C₂H₅ | OCHFCF₃ | F | |
| n-C₃H₇ | OCHFCF₃ | H | |
| n-C₃H₇ | OCHFCF₃ | F | |
| n-C₄H₉ | OCHFCF₃ | H | |
| n-C₄H₉ | OCHFCF₃ | F | |
| n-C₅H₁₁ | OCHFCF₃ | H | |
| n-C₅H₁₁ | OCHFCF₃ | F | |
| n-C₆H₁₃ | OCHFCF₃ | H | |
| n-C₆H₁₃ | OCHFCF₃ | F | |
| CH₃ | CF₃ | H | |
| CH₃ | CF₃ | F | |
| C₂H₅ | CF₃ | H | |
| C₂H₅ | CF₃ | F | |
| n-C₃H₇ | CF₃ | H | |
| n-C₃H₇ | CF₃ | F | |
| n-C₄H₉ | CF₃ | H | |
| n-C₄H₉ | CF₃ | F | |
| n-C₅H₁₁ | CF₃ | H | |
| n-C₅H₁₁ | CF₃ | F | |
| n-C₆H₁₃ | CF₃ | H | |
| n-C₆H₁₃ | CF₃ | F | |
| CH₃ | OCHFCHF₂ | H | |
| CH₃ | OCHFCHF₂ | F | |
| C₂H₅ | OCHFCHF₂ | H | |
| C₂H₅ | OCHFCHF₂ | F | |
| n-C₃H₇ | OCHFCHF₂ | H | |
| n-C₃H₇ | OCHFCHF₂ | F | |
| n-C₄H₉ | OCHFCHF₂ | H | |
| n-C₄H₉ | OCHFCHF₂ | F | |
| n-C₅H₁₁ | OCHFCHF₂ | H | |
| n-C₅H₁₁ | OCHFCHF₂ | F | |
| n-C₆H₁₃ | OCHFCHF₂ | H | |
| n-C₆H₁₃ | OCHFCHF₂ | F | |
| CH₃ | OC₂F₅ | H | |
| CH₃ | OC₂F₅ | F | |
| C₂H₅ | OC₂F₅ | H | |
| C₂H₅ | OC₂F₅ | F | |
| n-C₃H₇ | OC₂F₅ | H | |
| n-C₃H₇ | OC₂F₅ | F | |
| n-C₄H₉ | OC₂F₅ | H | |
| n-C₄H₉ | OC₂F₅ | F | |
| n-C₅H₁₁ | OC₂F₅ | H | |
| n-C₅H₁₁ | OC₂F₅ | F | |
| n-C₆H₁₃ | OC₂F₅ | H | |
| n-C₆H₁₃ | OC₂F₅ | F | |
| CH₃ | OC₃F₇ | H | |
| CH₃ | OC₃F₇ | F | |
| C₂H₅ | OC₃F₇ | H | |
| C₂H₅ | OC₃F₇ | F | |
| n-C₃H₇ | OC₃F₇ | H | K 65 S_{B} 157 l; Δn = +0.097; Δε = 26 |
| n-C₃H₇ | OC₃F₇ | F | |
| n-C₄H₉ | OC₃F₇ | H | |
| n-C₄H₉ | OC₃F₇ | F | |
| n-C₅H₁₁ | OC₃F₇ | H | |
| n-C₅H₁₁ | OC₃F₇ | F | |
| n-C₆H₁₃ | OC₃F₇ | H | |
| n-C₆H₁₃ | OC₃F₇ | F | |
| CH₃ | OCF=CF₂ | H | |
| CH₃ | OCF=CF₂ | F | |
| C₂H₅ | OCF=CF₂ | H | |
| C₂H₅ | OCF=CF₂ | F | |
| n-C₃H₇ | OCF=CF₂ | H | |
| n-C₃H₇ | OCF=CF₂ | F | |
| n-C₄H₉ | OCF=CF₂ | H | |
| n-C₄H₉ | OCF=CF₂ | F | |
| n-C₅H₁₁ | OCF=CF₂ | H | |
| n-C₅H₁₁ | OCF=CF₂ | F | |
| n-C₆H₁₃ | OCF=CF₂ | H | |
| n-C₆H₁₃ | OCF=CF₂ | F | |
| CH₃ | OCF₂CHFCF₃ | H | |
| CH₃ | OCF₂CHFCF₃ | F | |
| C₂H₅ | OCF₂CHFCF₃ | H | |
| C₂H₅ | OCF₂CHFCF₃ | F | |
| n-C₃H₇ | OCF₂CHFCF₃ | H | |
| n-C₃H₇ | OCF₂CHFCF₃ | F | |
| n-C₄H₉ | OCF₂CHFCF₃ | H | |
| n-C₄H₉ | OCF₂CHFCF₃ | F | |
| n-C₅H₁₁ | OCF₂CHFCF₃ | H | |
| n-C₅H₁₁ | OCF₂CHFCF₃ | F | |
| n-C₆H₁₃ | OCF₂CHFCF₃ | H | |
| n-C₆H₁₃ | OCF₂CHFCF₃ | F | |

### Beispiel 2

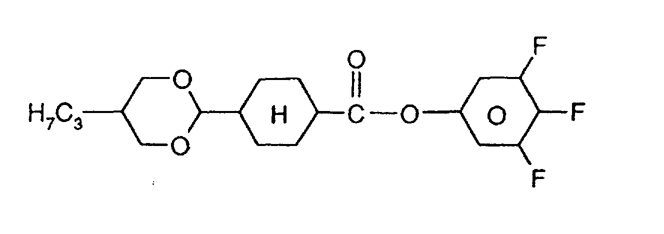

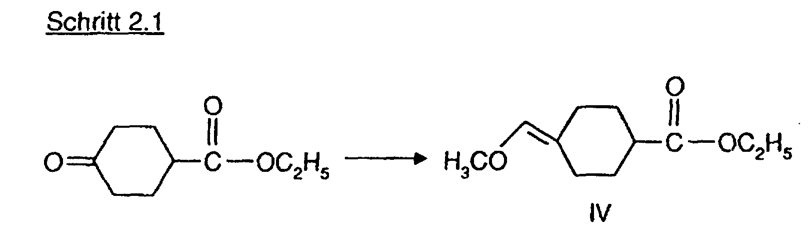

1,5 mol Cyclohexanoncarbonsäureethylester und 1,5 mol Methoxymethyltriphenylphosphoniumchlorid werden in 2 l Methyl-tert. butylether suspendiert und unter Kühlung bei -5 °C → +5 °C mit einer Lösung bestehend aus 1,5 mol Kalium-tert. butylat in 500 ml THF versetzt. Nach beendeter Zugabe wird über Nacht bei Raumtemperatur gerührt. Das Gemisch wird mit Wasser und 10 ml konz. HCl versetzt und anschließend wie üblich aufgearbeitet.

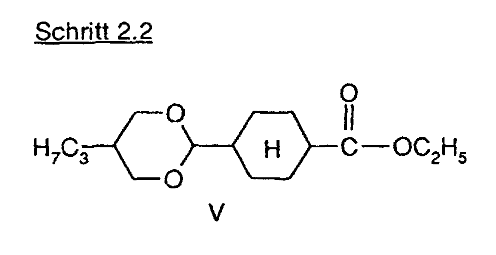

0,1 mol des Ethylethers IV, 0,1 mol Propylpropandiol, 2 g p-Toluolsulfonsäure und 100 ml Toluol werden 2 h am Wasserabscheider gekocht. Das Gemisch läßt man auf Raumtemperatur abkühlen und engt im Vakuum ein.

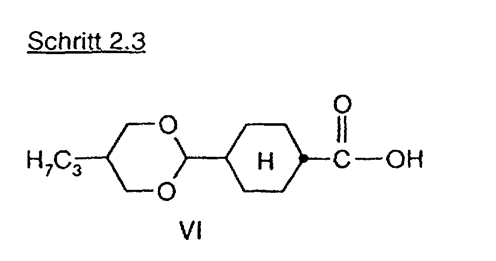

93 mmol Ester V, 0,1 mol KOH und 150 ml Methanol werden 24 h am Rückfluß gekocht. Man läßt auf Raumtemperatur abkühlen und versetzt unter Kühlung mit konz. HCI. Zuletzt wird wie üblich aufgearbeitet.

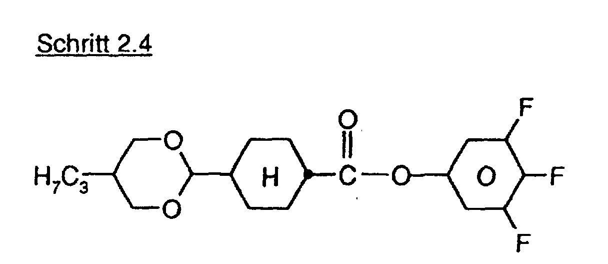

Analog Beispiel 1 werden 50 mmol VI, 55 mmol 3,4,5-Trifluorphenol, 55 mmol DCC und 225 mg DMAP in 75 ml Toluol über Nacht gerührt. Nach Zugabe von Essigester wird wie üblich aufgearbeitet. Das Produkt wird in Isopropanol umkristallisiert. K 73 S_{B} (51) N (63,8) l; Δn = +0,060; Δε = 22,46

Analog werden die folgenden Verbindungen der Formel 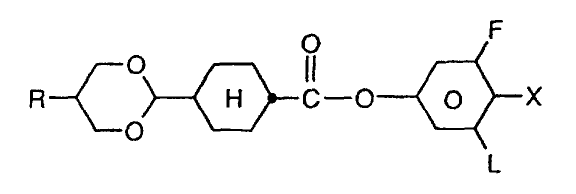 hergestellt:

| R | X | L | |
|---|---|---|---|
| CH₃ | F | H | |
| CH₃ | F | F | |
| C₂H₅ | F | H | K 81 S_{B} (66) N 82,5 l; Δn = +0,062; Δε = 17,7 |
| C₂H₅ | F | F | K 100 l; Δn = +0,044; Δε 27.4 |
| n-C₃H₇ | F | H | K 82 S_{B} 92 N 107,7 l; Δn = +0,065; Δε = 17 |
| n-C₄H₉ | F | H | |
| n-C₄H₉ | F | F | |
| n-C₅H₁₁ | F | H | K 69 S_{B} 90 N 111,6 l; Δn = +0,068; Δε = 15,3 |
| n-C₅H₁₁ | F | F | K 61 S_{B} (48) N 74,3 l; Δn = +0,061; Δε = 20.0 |
| n-C₆H₁₃ | F | H | |
| n-C₆H₁₃ | F | F | |
| CH₃ | Cl | H | |
| CH₃ | Cl | F | |
| C₂H₅ | Cl | H | |
| C₂H₅ | Cl | F | |
| n-C₃H₇ | Cl | H | |
| n-C₃H₇ | Cl | F | |
| n-C₄H₉ | Cl | H | |
| n-C₄H₉ | Cl | F | |
| n-C₅H₁₁ | Cl | H | |
| n-C₅H₁₁ | Cl | F | |
| n-C₆H₁₃ | Cl | H | |
| n-C₆H₁₃ | Cl | F | |
| CH₃ | OCF₃ | H | |
| CH₃ | OCF₃ | F | |
| C₂H₅ | OCF₃ | H | K 61 S_{B} 115 l**;** Δn = +0,065; Δε = 19,7 |
| C₂H₅ | OCF₃ | F | |
| n-C₃H₇ | OCF₃ | H | K 59 S_{B} 133 l; Δn = +0.069; Δε = 19,1 |
| n-C₃H₇ | OCF₃ | F | |
| n-C₄H₉ | OCF₃ | H | |
| n-C₄H₉ | OCF₃ | F | |
| n-C₅H₁₁ | OCF₃ | H | K 45 S_{B} 136 l; Δn = +0.070; Δε = 17,9 |
| n-C₅H₁₁ | OCF₃ | F | |
| n-C₆H₁₃ | OCF₃ | H | |
| n-C₆H₁₃ | OCF₃ | F | |
| CH₃ | CF₃ | H | |
| CH₃ | CF₃ | F | |
| C₂H₅ | CF₃ | H | |
| C₂H₅ | CF₃ | F | |
| n-C₃H₇ | CF₃ | H | |
| n-C₃H₇ | CF₃ | F | |
| n-C₄H₉ | CF₃ | H | |
| n-C₄H₉ | CF₃ | F | |
| n-C₅H₁₁ | CF₃ | H | |
| n-C₅H₁₁ | CF₃ | F | |
| n-C₆H₁₃ | CF₃ | H | |
| n-C₆H₁₃ | CF₃ | F | |
| CH₃ | CF₂H | H | |
| CH₃ | CF₂H | F | |
| C₂H₅ | CF₂H | H | |
| C₂H₅ | CF₂H | F | |
| n-C₃H₇ | CF₂H | H | |
| n-C₃H₇ | CF₂H | F | |
| n-C₄H₉ | CF₂H | H | |
| n-C₄H₉ | CF₂H | F | |
| n-C₅H₁₁ | CF₂H | H | |
| n-C₅H₁₁ | CF₂H | F | |
| n-C₆H₁₃ | CF₂H | H | |
| n-C₆H₁₃ | CF₂H | F | |
| CH₃ | OCH₂CF₃ | H | |
| CH₃ | OCH₂CF₃ | F | |
| C₂H₅ | OCH₂CF₃ | H | |
| C₂H₅ | OCH₂CF₃ | F | |
| n-C₃H₇ | OCH₂CF₃ | H | |
| n-C₃H₇ | OCH₂CF₃ | F | |
| n-C₄H₉ | OCH₂CF₃ | H | |
| n-C₄H₉ | OCH₂CF₃ | F | |
| n-C₅H₁₁ | OCH₂CF₃ | H | |
| n-C₅H₁₁ | OCH₂CF₃ | F | |
| n-C₆H₁₃ | OCH₂CF₃ | H | |
| n-C₆H₁₃ | OCH₂CF₃ | F | |
| CH₃ | OCH=CF₂ | H | |
| CH₃ | OCH=CF₂ | F | |
| C₂H₅ | OCH=CF₂ | H | |
| C₂H₅ | OCH=CF₂ | F | |
| n-C₃H₇ | OCH=CF₂ | H | |
| n-C₃H₇ | OCH=CF₂ | F | |
| n-C₄H₉ | OCH=CF₂ | H | |
| n-C₄H₉ | OCH=CF₂ | F | |
| n-C₅H₁₁ | OCH=CF₂ | H | |
| n-C₅H₁₁ | OCH=CF₂ | F | |
| n-C₆H₁₃ | OCH=CF₂ | H | |
| n-C₆H₁₃ | OCH=CF₂ | F | |
| CH₃ | OCHFCF₃ | H | |
| CH₃ | OCHFCF₃ | F | |
| C₂H₅ | OCHFCF₃ | H | |
| C₂H₅ | OCHFCF₃ | F | |
| n-C₃H₇ | OCHFCF₃ | H | |
| n-C₃H₇ | OCHFCF₃ | F | |
| n-C₄H₉ | OCHFCF₃ | H | |
| n-C₄H₉ | OCHFCF₃ | F | |
| n-C₅H₁₁ | OCHFCF₃ | H | |
| n-C₅H₁₁ | OCHFCF₃ | F | |
| n-C₆H₁₃ | OCHFCF₃ | H | |
| n-C₆H₁₃ | OCHFCF₃ | F | |
| CH₃ | CF₃ | H | |
| CH₃ | CF₃ | F | |
| C₂H₅ | CF₃ | H | |
| C₂H₅ | CF₃ | F | |
| n-C₃H₇ | CF₃ | H | |
| n-C₃H₇ | CF₃ | F | |
| n-C₄H₉ | CF₃ | H | |
| n-C₄H₉ | CF₃ | F | |
| n-C₅H₁₁ | CF₃ | H | |
| n-C₅H₁₁ | CF₃ | F | |
| n-C₆H₁₃ | CF₃ | H | |
| n-C₆H₁₃ | CF₃ | F | |
| CH₃ | OCHFCHF₂ | H | |
| CH₃ | OCHFCHF₂ | F | |
| C₂H₅ | OCHFCHF₂ | H | |
| C₂H₅ | OCHFCHF₂ | F | |
| n-C₃H₇ | OCHFCHF₂ | H | |
| n-C₃H₇ | OCHFCHF₂ | F | |
| n-C₄H₉ | OCHFCHF₂ | H | |
| n-C₄H₉ | OCHFCHF₂ | F | |
| n-C₅H₁₁ | OCHFCHF₂ | H | |
| n-C₅H₁₁ | OCHFCHF₂ | F | |
| n-C₆H₁₃ | OCHFCHF₂ | H | |
| n-C₆H₁₃ | OCHFCHF₂ | F | |
| CH₃ | OC₂F₅ | H | |
| CH₃ | OC₂F₅ | F | |
| C₂H₅ | OC₂F₅ | H | |
| C₂H₅ | OC₂F₅ | F | |
| n-C₃H₇ | OC₂F₅ | H | |
| n-C₃H₇ | OC₂F₅ | F | |
| n-C₄H₉ | OC₂F₅ | H | |
| n-C₄H₉ | OC₂F₅ | F | |
| n-C₅H₁₁ | OC₂F₅ | H | |
| n-C₅H₁₁ | OC₂F₅ | F | |
| n-C₆H₁₃ | OC₂F₅ | H | |
| n-C₆H₁₃ | OC₂F₅ | F | |
| CH₃ | OC₃F₇ | H | |
| CH₃ | OC₃F₇ | F | |
| C₂H₅ | OC₃F₇ | H | |
| C₂H₅ | OC₃F₇ | F | |
| n-C₃H₇ | OC₃F₇ | H | |
| n-C₃H₇ | OC₃F₇ | F | |
| n-C₄H₉ | OC₃F₇ | H | |
| n-C₄H₉ | OC₃F₇ | F | |
| n-C₅H₁₁ | OC₃F₇ | H | |
| n-C₅H₁₁ | OC₃F₇ | F | |
| n-C₆H₁₃ | OC₃F₇ | H | |
| n-C₆H₁₃ | OC₃F₇ | F | |
| CH₃ | OCF=CF₂ | H | |
| CH₃ | OCF=CF₂ | F | |
| C₂H₅ | OCF=CF₂ | H | |
| C₂H₅ | OCF=CF₂ | F | |
| n-C₃H₇ | OCF=CF₂ | H | |
| n-C₃H₇ | OCF=CF₂ | F | |
| n-C₄H₉ | OCF=CF₂ | H | |
| n-C₄H₉ | OCF=CF₂ | F | |
| n-C₅H₁₁ | OCF=CF₂ | H | |
| n-C₅H₁₁ | OCF=CF₂ | F | |
| n-C₆H₁₃ | OCF=CF₂ | H | |
| n-C₆H₁₃ | OCF=CF₂ | F | |
| CH₃ | OCF₂CHFCF₃ | H | |
| CH₃ | OCF₂CHFCF₃ | F | |
| C₂H₅ | OCF₂CHFCF₃ | H | |
| C₂H₅ | OCF₂CHFCF₃ | F | |
| n-C₃H₇ | OCF₂CHFCF₃ | H | |
| n-C₃H₇ | OCF₂CHFCF₃ | F | |
| n-C₄H₉ | OCF₂CHFCF₃ | H | |
| n-C₄H₉ | OCF₂CHFCF₃ | F | |
| n-C₅H₁₁ | OCF₂CHFCF₃ | H | |
| n-C₅H₁₁ | OCF₂CHFCF₃ | F | |
| n-C₆H₁₃ | OCF₂CHFCF₃ | H | |
| n-C₆H₁₃ | OCF₂CHFCF₃ | F | |
| n-C₃H₇ | OCHFCHF₂ | H | |
| n-C₃H₇ | OCHFCHF₂ | F | |
| n-C₅H₁₁ | OCHFCHF₂ | H | |
| n-C₅H₁₁ | OCHFCHF₂ | F | |
| CH₂=CH | F | H | |
| CH₂=CH | F | F | |
| CH₃-CH=CH | F | H | |
| CH₃-CH=CH | F | F | |
| CH₂=CH-C₂H₄- | F | H | |
| CH₂=CH-C₂H₄- | F | F | |
| CH₃CH=CH-C₂H₄ | F | H | |
| CH₃CH=CH-C₂H₄ | F | F | |

### Mischungsbeispiele

### Beispiel A

| | | |
|---|---|---|
| PCH-5F | 9,50 % | Klärpunkt [°C]: 92,5 |
| PCH-6F | 7,60 % | Δn [589 nm, 20 °C]: 0,0977 |
| PCH-7F | 5,70 % | Δε [1 kHz, 20 °C]: 7,34 |
| CCP-2OCF₃ | 7,60 % | |
| CCP-3OCF₃ | 11,40% | |
| CCP-4OCF₃ | 8,55 % | |
| CCP-5OCF₃ | 8,55 % | |
| BCH-3F.F | 11,40 % | |
| BCH-5F.F | 9,50 % | |
| ECCP-3OCF₃ | 4,75 % | |
| ECCP-5OCF₃ | 4,75 % | |
| CBC-33F | 1,90 % | |
| CBC-53F | 1,90 % | |
| CBC-55F | 1,90 % | |
| DPZU-3F | 5,00 % | |

### Beispiel B

| | |
|---|---|
| PCH-5F | 9,50 % |
| PCH-6F | 7,60 % |
| PCH-7F | 5,70 % |
| CCP-2OCF₃ | 7,60 % |
| CCP-3OCF₃ | 11,40 % |
| CCP-4OCF₃ | 8,55 % |
| CCP-5OCF₃ | 8,55 % |
| BCH-3F.F | 11,40 % |
| BCH-5F.F | 9,50 % |
| ECCP-3OCF₃ | 4,75 % |
| ECCP-5OCF₃ | 4,75 % |
| CBC-33F | 1,90 % |
| CBC-53F | 1,90 % |
| CBC-55F | 1,90 % |
| DCZU-3-F | 5,00 % |

## Patentansprüche

1. 1,3-Dioxane der Formel I, 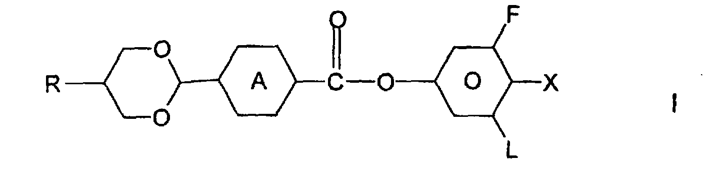 worin
R H, einen unsubstituierten, einen einfach durch CN oder CF₃ oder einen mindestens einfach Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S- 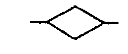 -CO-, -CO-O-, -O-CO- oder -O-CO-O- so ersetzt sein können, daß O-Atome nicht direkt miteinander verknüpft sind, 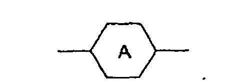
(a) trans-1,4-Cyclohexylen,
(b) 1,4-Phenylen, worin auch ein oder zwei CH-Gruppen durch N ersetzt sein können,
(c) 1,4-Cyclohexenylen,
wobei die Reste (b) und (c) ein- oder mehrfach durch Fluor substituiert sein können.
X F, Cl, halogeniertes Alkyl, Alkoxy oder Alkenyl mit 1 - 5 C-Atomen, und
L H oder F
bedeuten,
mit der Maßgabe, daß
im Fall X = F oder X = Cl L Fluor bedeutet.

2. 1,3-Dioxane der Formel l1, 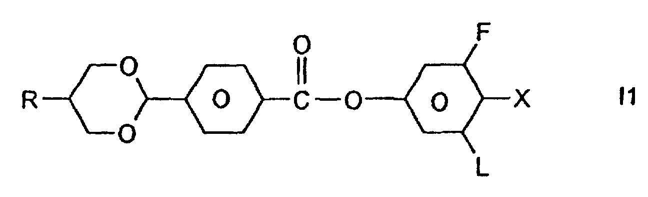 worin R, X und L die in Anspruch 1 angegebene Bedeutung haben.

3. 1,3-Dioxane der Formel 12, 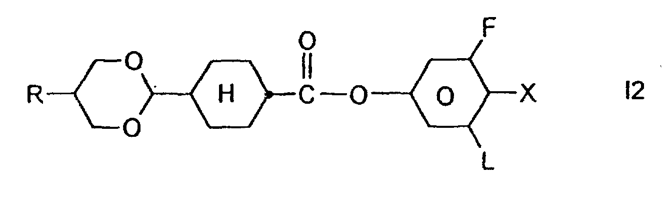 worin R, X und L die in Anspruch 1 angegebene Bedeutung haben.

4. 1,3-Dioxane nach einem der Ansprüche 1 bis 3, **dadurch gekennzeichnet, daß** X F, Cl, OCF₃, OCHF₂, OCH₂CF₃, OCHFCF₃, OCH=CF₂, OCF=CF₂ oder OC₂F₅ bedeutet.

5. 1,3-Dioxane nach einem der Ansprüche 1 bis 4, **dadurch gekennzeichnet, daß** R ein geradkettiger Alkyl- oder Alkenylrest mit 1 bis 5 C-Atomen ist.

6. 1,3-Dioxane nach einem der Ansprüche 1 bis 5, **dadurch gekennzeichnet, daß** X = L = F ist.

7. Verwendung von Verbindungen der Formel I als Komponenten flüssigkristalliner Medien.

8. Flüssigkristallines Medium mit mindestens zwei flüssigkristallinen Komponenten, **dadurch gekennzeichnet, daß** es mindestens eine Verbindung der Formel I enthält.

9. Flüssigkristall-Anzeigeelement, **dadurch gekennzeichnet, daß** es ein flüssigkristallines Medium nach Anspruch 8 enthält.

10. Elektrooptisches Anzeigeelement, **dadurch gekennzeichnet, daß** es als Dielektrikum ein flüssigkristallines Medium nach Anspruch 8 enthält.

## Claims

1. 1,3-Dioxanes of the formula I 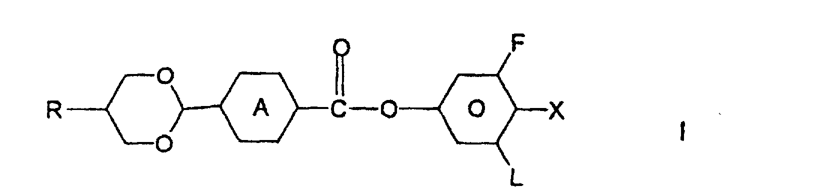 in which
R is H, an alkyl or alkenyl radical having from 1 to 15 carbon atoms which is unsubstituted, monosubstituted by CN or CF₃ or at least monosubstituted by halogen, where, in addition, one or more CH₂ groups in these radicals may each, independently of one another, be replaced by -O-, -S-, 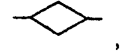 -CO-, -CO-O-, -O-CO- or -O-CO-O- in such a way that O atoms are not linked directly to one another, 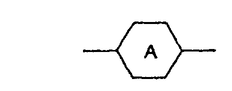 is
(a) trans-1,4-cyclohexylene,
(b) 1,4-phenylene, in which, in addition, one or two CH groups may be replaced by N,
(c) 1,4-cyclohexenylene,
where the radicals (b) and (c) may be monosubstituted or polysubstituted by fluorine,
X is F, Cl, halogenated alkyl, alkoxy or alkenyl having 1-5 carbon atoms, and
L is H or F,
with the proviso that
in the case where X = F or X = Cl, L is fluorine.

2. 1,3-Dioxanes of the formula l1 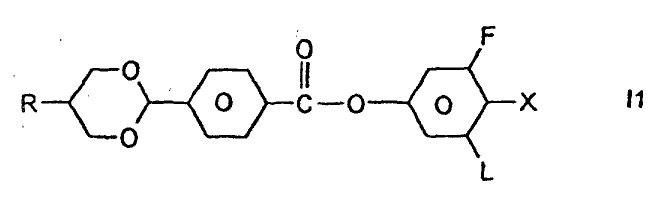 in which R, X and L are as defined in Claim 1.

3. 1,3-dioxanes of the formula l2 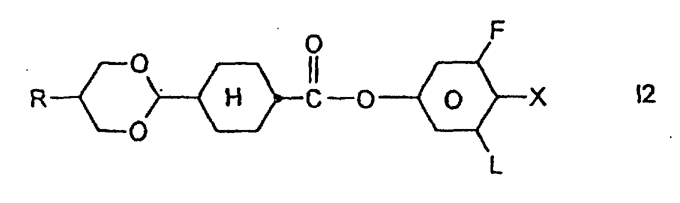 in which R, X and L are as defined in Claim 1.

4. 1,3-Dioxanes according to one of Claims 1 to 3, **characterised in that** X is F, Cl, OCF₃, OCHF₂, OCH₂CF₃, OCHFCF₃, OCH=CF₂, OCF=CF₂ or OC₂F₅.

5. 1,3-Dioxanes according to one of Claims 1 to 4, **characterised in that** R is a straight-chain alkyl or alkenyl radical having from 1 to 5 carbon atoms.

6. 1,3-Dioxanes according to one of Claims 1 to 5, **characterised in that** X = L = F.

7. Use of compounds of the formula I as components of liquid-crystalline media.

8. Liquid-crystalline medium having at least two liquid-crystalline components, **characterised in that** it comprises at least one compound of the formula I.

9. Liquid-crystal display element, **characterised in that** it comprises a liquid-crystalline medium according to Claim 8.

10. Electro-optical display element, **characterised in that** it contains, as dielectric, a liquid-crystalline medium according to Claim 8.

## Revendications

1. 1,3-dioxans de la formule I 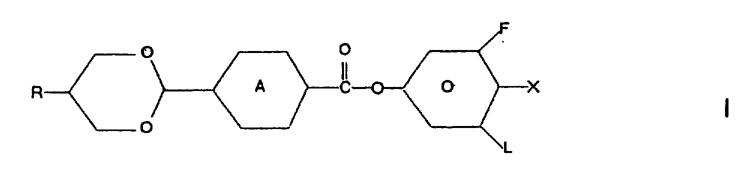 dans laquelle
R représente H, un radical d'alkyle ou d'alcényle avec 1 à 15 atomes C, non substitué ou monosubstitué par CN ou CF₃ ou au moins monosubstitué par halogène, où dans ces radicaux un ou plusieurs groupes CH₂ peuvent être substitués indépendamment par -O-, -S- 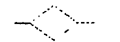 -CO-, -CO-O-, -O-CO- ou -O-CO-O-, de manière à ce que les atomes O ne soient pas liés directement entre eux. 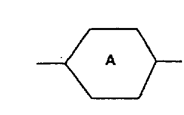
(a) représente trans-1,4-cyclohexylène,
(b) 1,4-phénylène, où un ou deux groupes CH peuvent être remplacés par N,
(c) 1,4-cyclohexénylène,
où les radicaux (b) et (c) peuvent être mono- ou multisubstitués par du fluor,
X représente F, Cl, alkyle halogéné, alkoxy halogéné ou alcényle halogéné avec 1 à 5 atomes C, et
L représente H ou F,
dans la mesure où :
si X = F ou X = Cl, L représente du fluor.

2. 1,3-dioxans de la formule l1, 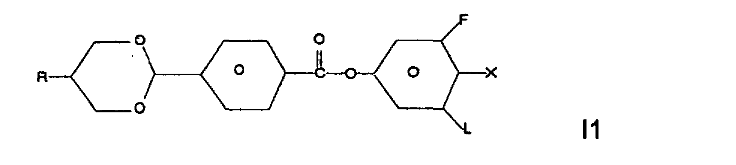 dans laquelle R, X et L ont la signification indiquée dans la revendication 1.

3. 1,3-dioxans de la formule I2, 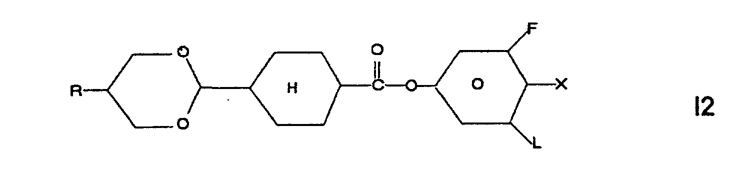 dans laquelle R, X et L ont la signification indiquée dans la revendication 1.

4. 1,3-dioxans selon l'une des revendications 1 à 3, **caractérisés en ce que** X représente F, Cl, OCF₃, OCHF₂, OCH₂CF₃, OCHFCF₃, OCH=CF₂, OCF=CF₂ ou OC₂F₅.

5. 1,3-dioxans selon l'une des revendications 1 à 4, **caractérisés en ce que** R est un radical linéaire d'alkyl ou d'alcényle ayant 1 à 5 atomes C.

6. 1,3-dioxans selon l'une des revendications 1 à 5, **caractérisés en ce que** X = L = F.

7. Utilisation de combinaisons de la formute I en tant que composants de médias de cristaux liquides.

8. Médium de cristaux liquides ayant au moins deux composants de cristaux liquides, **caractérisé en ce qu'**il comporte au moins une combinaison de la formule I.

9. Elément d'affichage à cristaux liquides, **caractérisé en ce qu'**il contient un médium de cristaux liquides selon la revendication 8.

10. Elément d'affichage électro-optique, **caractérisé en ce qu'**il contient, en tant que diélectrique, un médium de cristaux liquides selon la revendication 8.
